# EUROPEAN PATENT APPLICATION

(11) **EP 4 253 942 A1**
(43) Date of publication of application: **04.10.2023**
(21) Application number: 22166077.2
(22) Date of filing: 31.03.2022
(51) Int. Cl.: G01N 21/64

(54) **METHOD AND APPARATUS FOR OPTICAL IMAGING WITH A HIGH NUMBER OF IMAGING SAMPLES**

(71) Applicant: Abberior GmbH, 37077 Göttingen (DE)
(72) Inventor: DONNERST, Gerald, 37077 Göttingen (DE); HELL, Stefan W., 37085 Göttingen (DE)
(74) Representative: Kröncke, Rolf

(57) **Abstract**

In a first aspect, the invention relates to a method for imaging and screening a component of interest in a sample based on different types of imaging. In particular, the method comprises first acquiring low or high magnification images of the sample and, after determining the region of interest, MINFLUX and/or MINSTED superresolution microscopy of the region of interest to screen the compound of interest. A system is further described for imaging and screening a compound of interest in a sample, the system comprising a microscope system combining diffraction-limited imaging techniques with at least one of MINFLUX or MINSTED superresolution techniques. The system optionally comprises a database of properties of known compounds. Finally, a computer program product resided on a computer-readable medium is provided to enable the method of the invention to be carried out for controlling a microscope system.

## Description

In a first aspect, the invention relates to a method for imaging and screening a component of interest in a sample based on different types of imaging. In particular, the method comprises first acquiring low or high magnification images of the sample and, after determining the region of interest, MINFLUX and/or MINSTED superresolution microscopy of the region of interest to screen the compound of interest. A system is further described for imaging and screening a compound of interest in a sample, the system comprising a microscope system combining diffraction-limited imaging techniques with at least one of MINFLUX or MINSTED superresolution techniques. The system optionally comprises a database of properties of known compounds. Finally, a computer program product resided on a computer-readable medium is provided to enable the method of the invention to be carried out for controlling a microscope system.

### Prior art

Microscopy is a common tool for imaging objects and observing compounds i*n situ*. Optical microscopes are the tool of choice for visualising the structures and dynamic processes inside cells or in virtually any specimen. In particular, optical microscopes allow the visualisation of processes *in vivo* in cells and are therefore frequently used in biomedicine to identify relevant structures for the diagnosis and treatment of diseases, but also for drug development.

Normal optical microscopy is limited in view of the diffraction of light. New technologies, the so called superresolution technology, have been developed to circumvent the resolution limit while maintaining the minimally invasive nature of optical microscopy.

Optical microscopy and, in particular, superresolution microscopy came into focus for imaging molecules at the nanoscale level. In particular, its use in observing movement in living cells is described. Studying the influence of molecules on said movement is possible with superresolution microscopy and, consequently, it is speculated that superresolution microscopy may aid drug discovery.

Drug discovery and development uses drug screening and other methods that examine a variety of substances for their ability to interact with other substances and structures and alter functionalities in cells are used for drug. With microscopy aid, it is possible to examine interaction of molecules with target molecules in biological objects and, in addition, to observe resulting reactions of the substances on the target molecules or objects over time. However, the overall number of reactions to be monitored, e. g. by microscopy, is typically a multiple of the number of substances to be searched since, on the one hand, several identical pairs are necessary to get statistically significant results and, on the other hand, substances should often have the desired properties in relation to different other substances or different biological objects. Reactions on a molecular level can often only be ascertained by means of analytical reactions. Thus, monitoring reaction dynamics is often not possible at all. In particular, currently available embodiments using optical microscopes are limited either in terms of the number of compounds to be observed or limited resolution, thus, no specific effects can be determined.

Drug screening targets include cancer targets, like T-cell engagers, neurological targets, infection targets, metabolism targets, autoimmune targets, inflammation targets, renal targets, cardiovascular targets and pain targets. However, it is desired to observe and screen these targets in living materials, like living cells. In addition, time resolved observation, i.e. monitoring reaction dynamics, should be possible.

Fluorescence microscopy recently experienced a second resolution boost through the synergistic combination of the specific strength of the coordinate-oriented superresolution family represented by STED and RESOLFT and its coordinate-stochastic counterpart comprising PALM/STORM and PAINT. The resulting concept, called Minimal Photon Fluxes (MINFLUX), closes the prevailing resolution gap of about 20 to 30 nm in STED, PALM/STORM and other fluorescent nanoscopes to the order of 1 to 5 nm of the molecules themselves. The MINFLUX principle enables the precise localisation of a fluorescence marker through a donut-shaped focus of a laser beam used to excite the fluorescence. As long as the fluorescence marker is within the donut ring, but not exactly at its zero point, it emits fluorescence photons that can be detected. If the donut-shaped focus is now placed at at least three illumination points of an illumination point pattern around the fluorescence marker, the position of the fluorescence marker can be determined using a type of triangulation method. The so-called MINSTED microscopy is a combination of STED microscopy with the triangulation method known from MINFLUX microscopy to obtain corresponding images and described in Weber et al, "MINSTED fluorescence localization and nanoscopy". Nature Photonics 15, 361-366 (2021) and Weber et al, doi: https://doi.org/10.1101/2022.03.18.484906.

Here, the excitation light is used to excite fluorescence in combination with a fluorescence inhibition light, namely a light for generating stimulated fluorescence, e.g. a STED light that suppresses free fluorescence. In this case, the fluorescence inhibition light is preferably focused in such a way that the focus is at such a minimum that the stimulation light can be focused, as is it conventional, for example, in confocal fluorescence microscopy. Both methods, MINFLUX and MINSTED, are used to localise structures well below the diffraction limit and beyond, with increased resolution compared to STED/RESOLFT microscopy. Because MINFLUX localisation is no longer limited by waiting for a large number of fluorescence photons, this nanometre-accurate localisation is much faster than the "passive" camera-based localisation used in PALM/STORM.

Although various methods are described, including optical microscopes for drug screening, it is desired to provide further methods and systems allow high throughput drug screening, while observing the drug targets on nanoscale.

### Brief description of the present invention

In a first aspect, the presenr invention relates to a method of image sequences for imaging and screening a component of interest in a sample, comprising the steps of:
a) Providing a sample, like living material, having the component of interest whereby the compound of interest is labelled with a first detection label and, optionally, the sample is labelled with at least one further detection label;
b) Obtaining at least one low magnification image and/or at least one high magnification image of the sample by diffraction limited imaging with a device;
c) Determining a first region of interest of said sample based on the low and/or high magnification image of step b);
d) Optionally determining further at least one second region of interest of said sample based on the low and/or high magnification image of the sample obtained in step b);
e) Obtaining and imaging the component of interest in at least the first region of interest by low and/or high magnification imaging by diffraction limited imaging;
f) Optionally redetermining at least one of the regions of interest;
g) Screening the component of interest in the at least first region of interest by MINSTED and/or MINFLUX superresolution microscopy;
h) Imaging and/or tracking the component of interest in the sample and determining information of the component of interest in the sample based on steps e) and g), in particular, image sequences information.

The present inventors have recognized that combining an optical microscopy technology based on diffraction limited imaging to obtain low magnification and/or high magnification overview images of a sample and based thereon determining at least one region of interest, using MINFLUX or MINSTED superresolution microscopy to examine specific compounds. In particular, by using intelligent algorithms it is possible to define said regions of interest and, simultaneously to image or track the compounds of interest in the regions of interest, thereby, enabling dynamic examination of said compounds and the effects of said compounds over time in the sample.

As used herein, the sample comprising the component of interest includes living or non-living material. In an embodiment, the components of interest are evaluated in living material, like living cells, over time. In particular, as described for superresolution techniques, it is possible to observe influence of said compound of interest on various dynamic events in the cells including binding, binding stability, binding kinetics, steric orientation of bound compound, location of binding site, proximity of compound to binding site.

As used herein, the term "imaging" refers to a spatial determination of the components and structures. This typically includes identifying of the same macromolecular region. That is, in image sequences the images identify the same region at different times.

As used herein, the term "tracking" refers to image sequences in which a predefined target is tracked over time, i.e., the tracking is defined by the spatial and temporal tracking of the predetermined component.

In particular, it is possible to observe the spatial distribution of said compounds and the tracking of the movement of said compounds. Moreover, it is possible to examine and track the interaction of the compound of interest with other compounds of interest. In particular, the interaction between the compound of interest and at least a second known compound can be observed when the second compound is labelled with a different label. A typical example of interaction is the formation of binding pairs composed of a least one of a first binding partner and at least one of a second binding partner. Binding pairs include receptor-ligand binding pairs, enzyme homotropic or heterotropic effectors, etc.

The present invention allows for earlier and faster decision-making on candidate substances. This saves time in screening. Moreover, better decisions can be made on substances and their chemical modifications. This will lead to a better drug development, which in turn will lead to better drug development outcomes i.e. better drugs with a better and educated effect on the targeted disease.

In particular, the method of the present invention as well as the system according to the present invention allow for a more detailed and, thus, superior observation and, consequently, data of the respective components in terms of both superresolution as well as motion tracking.

As used herein, the terms "component of interest" and "compound of interest" are used interchangeably, unless otherwise indicated.

As used herein, "detection label" and "detection marker" are used interchangeably herein, unless otherwise indicated.

As used herein, the term "diffraction limited microscopy" or "diffraction limited imaging techniques" includes known techniques like widefield microscopy, confocal microscopy as well as light sheet microscopy, structured microscopy, illuminated or epifluorescence microscopy.

The term "sequences of images" or "image sequences" refers to sequential images that allows time resolved analysis of the component under investigation. The time scale of the sequences of images may be milliseconds, seconds, minutes, hours or even days.

That is, according to the present invention the method of sequences of imaging and screening of a compound of interest in a sample comprises in a first step imaging of the sample with low and high magnification being a diffraction limited imaging technique, while at least a first superresolution microscopy step follows, whereby the superresolution microscopy is performed with at least a first region of interest determined based on the images of the diffraction limited imaging. According to the method of the present invention, a sample with the compound of interest is provided. The compound of interest present in the sample is labelled with a first detected detection label. In addition, according to an embodiment the sample or parts of the sample are labelled additionally with at least one further detection label.

The component of interest is labelled by known means. Said labelling includes a labelling with a detection label, like antibody labelling, labelling by click chemistry, snaptag or halotag labelling, nanobody labelling, DNA hybridization as in the method called PAIN, labelling of targets one by one by imaging as well as labelling by scattering particles including metals and noble metals, like Au, Ag, quantum dots or PT.

In addition, the further detection label may be any label, e. g. intercalating DNA label or the like, but also chemiluminescence or luminescence label, allowing detection of specific structures of a sample. These structures include DNA or other nucleic acid structures, but also protein structures, organelle structures and the like. That is, based on i) the label and/or ii) the images obtained by diffraction limited imaging, structural patterns in the sample including organelles within the cells, like cell nuclei, mitochondria, the endoplasmic reticulum, the ribosomes, or the Golgi apparatus but also other elements or compounds within the cells can be determined. In an embodiment, the determination of the specific structural patterns is performed by applying intelligent algorithm (artificial intelligence), like artificial neural network.

In a next step, the at least one low magnification image or at least one high magnification image is obtained with a device, typically an optical microscope. As said, the low or high magnification is obtained by diffraction limited imaging. Based on this image, a first region of interest is determined. The determination is based on the detection of at least one of the detection labels or detection markers and, optionally, of structural patterns present in said sample.

By first applying the diffraction limited imaging and then determining the first region of interest, it is possible to reduce the data set obtained and to speed up the high throughput screening process.

In an embodiment, at least a second or further region of interest may be determined in said sample.

The determination of the regions of interest may be performed using intelligent algorithm, like artificial neural networks and other artificial intelligence in general. After determination of the region of interest, the further (sequences of) images with low and/or high magnification are obtained based on diffraction limited imaging and, if necessary, the at least first or the at least second region of interest is redetermined.

In the next step, the component of interest in the at least first region of interest is imaged and screened by MINFLUX and/or MINSTED superresolution microscopy. That is, MINFLUX and/or MINSTED superresolution microscopy means that screening and imaging is effected at nanoscale dimension. The MINFLUX and/or MINSTED superresolution microscopy according to the present invention includes a resolution/precision of at least 10 nm, e.g. 5 nm, like 2 nm, in particular, 1 nm resolution.

In particular, screening in the selected region focussing on the selected compound of interest allows to reduce the amount of data obtained and to process it faster. Furthermore, tracking the component of interest is advantageous as it is possible to determine the movement and tracking as well as monitoring reaction dynamics at nanoscale level.

The method additionally comprises determining time resolved (sequence of images) information, based on the image sequences of the compound of interest in the sample.

In an embodiment of the present invention, at least some of the steps are iterated. Of note, it may not be required to conduct all steps but only some of the steps. Namely, the superresolution screening for determining information and data may not be performed as the final iterative step but information and data may be obtained from the imaging step of the low and/or high magnification images obtaining e.g. by diffraction limited imaging (step e) and h)). Further, in embodiments of the present invention, that may include obtaining low and/or high magnification first imaging with a first time resolution of a first cycle, e.g. of milliseconds, while superresolution imaging as a second type of imaging may be performed in with a different reiteration, e.g. every 5^{th} time of the first imaging and with a different time cycle, e.g. in seconds. That is, this enables to reduce the data set or data volume as well as allowing quicker results in high throughput screening. For example, sometimes it is sufficient to observe a phenomenon by low and/or high magnification imaging and to determine interaction of components and tracking of components by superresolution occasionally. It may be sufficient to identify the mechanism of action of the component of interest by MINFLUX and/or MINSTED superresolution, followed by further imaging and/or tracking by diffraction limited imaging (step e)) only. This is particularly valuable when comparing the data and information with data sets present in a database.

The term "mechanism of action" as used herein refers to the specific biochemical interaction through which a candidate substance produces a desired pharmacological or functional effect. The interaction include interaction with binding partners, interaction with target structures including enzymes, for example altering a property of the target structure, etc. as well as co-localization with target structures in drug screening.

In an embodiment, the iteration may include the first imaging by diffraction limited imaging and the second step of first superresolution imaging if applicable, and not including the second superresolution imaging as described in the following. Alternatively, each of the first, second and third imaging can be at a different rate, e.g. for reducing the data set and pace up the method.

In an embodiment of the present invention, an at least second labelled compound is present, which compound may be a different compound than the compound of interest. That is, relative movement and tracking including possible interactions between the compound of interest and the at least second compound can be imaged and tracked. The information obtained based on the initial data from the superresolution microscopy in connection with the imaging by diffraction limited imaging allows to identify location of the compound of interest as well as the interaction and dynamics of said compound of interest over time.

The Superresolution can improve current drug screening on two independent levels: whereas with conventional drug screening results in several hundreds of hits for several 100000 targets, the benefits of superresolution data for drug screening allows earlier and faster drug decision, as superresolution data allow monitoring and tracking of the locally resolved determination of the compound of interest. In particular, when looking for interactions with a second compound of the sample or with a second compound or component added to the sample, therefore, it is possible to gain significant better superresolution imaging and tracking data, which means a better understanding of the components of interest, interaction with other components of the sample as well as further understanding of the components and binding characteristics with other molecules can be obtained. it is possible to determine binding and motion of the component of interest. This may be of interest in case of receptor-ligand binding or allosteric changes of enzymes and transport through membranes etc.

Based on this improvement in the first step of drug screening, the second step of screening based on the initial hits in the target molecules, typically several hundred hits, superresolution screening of all hits is possible. On this basis, better decisions on substances and eventually the chemical modifications is possible. That is, based on the data obtained by superresolution of the selected hits, it is possible to obtain additional information on the compounds of interest, especially, interaction with other compounds. These other compounds may be a component externally added to the sample or compounds present in the sample.

In another round, the discovered lead molecules, can be analysed further to arrive at candidate molecules. Based on the superresolution screening of the compounds, faster and more precise information is obtained, thus, allowing more successful decisions to be made about lead and candidate compounds.

In particular, based on the method according to the present invention including the MINFLUX and/or MINSTED superresolution screening in combination with the diffraction limited imaging, makes it possible to arrive at lead or even candidate compounds in one screening process.

In an embodiment of the present invention, the superresolution microscopy may include two different types of superresolution microscopy. Namely, a first type of superresolution microscopy having a lower resolution, e. g. from about 50 to 10 nm, which allows to analyse a larger number of compounds of interest or different compounds of interest. In a further step, a second type of superresolution microscopy is used, namely, MINSTED or MINFLUX which allows for a higher resolution of about 1-10 nm.

That is, in an embodiment of the present invention the method comprises three imaging methods, namely, a first diffraction limited imaging method, followed by a first and eventually second superresolution microscopy imaging method, whereby the second superresolution microscopy imaging method is one of MINSTED or MINFLUX. The first superresolution microscopy imaging method as used in these three imaging technique based method is selected from PALM, STORM, single molecule localisation microscopy, PAINT, DNA PAINT, and STED.

In an embodiment, the method according to the present invention is a method wherein the determination of the first region of interest, the redetermination and the imaging and screening comprises the use of intelligent algorithm, e. g. artificial neural networks to identify the one or more regions of interest within the low or high magnification image of the steps according to the present invention.

For example, by using the artificial neural network, specific structural pattern in the sample can be determined, or, in addition or alternatively, specific compounds like protein or protein complexes.

In particular, the embodiment with three imaging techniques, namely, the diffraction limited imaging with a first superresolution imaging, e. g. PAINT, or PAL/STORM and a second superresolution imaging of MINSTED and/or MINFLUX enables to determine a region of interest, then, based on the coordinate-stochastic technique by e.g. PALM/STORM followed by coordinate-target superresolution of MINSTED and MINFLUX, allowing specific tracking and screening of (single) component of interest. The combination of resolutions, the diffraction limited resolution with the first superresolution having a resolution at most 10 nm and the MINFLUX/MINSTED superresolution 1nm, enables to obtain fast and reliable information on the nanoscale below 5 nm of interaction tracking and coordination with other components as well as dynamic movement while tracking the component of interest. This is beneficial for electing the desired components of interest, e. g. in the field of drug screening allowing for speeding up the high throughput screening process in drug screening significantly. The imaging, screening and tracking according to the present invention is supported with intelligent algorithms, such as the artificial neural network mentioned.

The term "intelligent algorithm" and "artificial intelligence" are used herein interchangeably.

Further, the term "artificial intelligence" or "artificial neural network" Includes embodiments of among others, e.g. U-Net's, fully convolutional networks (FCN), feature pyramid networks (FPN), conventional neural networks (CNN), attention-based models as well as generative adversarial networks (GAN).

In addition, the method of sequence of images imaging and screening a compound of interest in a sample is a method wherein the compound of interest is labelled with a detection label being a fluorescent label.

The fluorescence marker or fluorescence label when used for different structures or different compounds is selected from fluorescent markers which differ in their respective properties, wherein individual fluorescent markers may be selectively excited for excitation light of different wavelengths or fluorescence light omitted by the way of wavelength selected way. Further, these fluorescence markers may be selected from fluorescence markers having an active state in which they can be excited by excitation light to emit fluorescence light and in an inactive state in which they, at least with the same excitation light, cannot be excited for the emission of the fluorescence light, and that can be transferred between the active and the inactive state. Depending on the type of technique applied for superresolution microscopy, only one fluorescent marker may be left in its active state or brought in its active state, which can be done selectively or on the basis of transfer probabilities, so that the registered fluorescent light following the excitation with the excitation light may only originate from that one. Particularly, fluorescent markers may thus be selected from those fluorescence markers which differ in at least one way on the excitation spectrum and their emission spectrum, or which can be switched with switching light out of their active state in their inactive state. The switching light can generally have the same wavelength as the excitation light or the further light which has an influence on the emission of fluorescent light by the fluorescent marker.

The skilled person is well aware of suitable fluorescent markers in the application of the respective technique, e. g. of PAINT or MINSTED or MINFLUX. The skilled person is well aware of suitable fluorescent label molecules, in particular, allowing the simultaneous detection and determination of different fluorescent labels at the same time. For example, suitable fluorescent labels include photoactivatable and photoswitchable fluorophores, such as the commercially available Abberior Cage and Abberior Flip dyes, and photoactivatable fluorescence proteins like mMaple, and mEos, and photoswitchable fluorescence proteins like those called Dronpa and Dreiklang.

As noted, the skilled person is well aware of the detection label, typically a fluorescent label, to be used for labelling the compound of interest. The labelling technology is known to the skilled person well. Various possibilities are known to the skilled person.

As said, the superresolution microscopy encompasses various types of superresolution imaging. In short: the PALM/STORM technique is based on blinking fluorescent molecules, i.e. molecules with a certain time "on" (i.e. flurescent) and a certain time ,off'. Both states can be triggered by different stimulus, e.g. via heat (i.e. spontaneous) or e.g. light induced. PALM/STORM is typically arranged in a widefield arrangement while the fluorescent emission of each molecule in the 'on' state is imaged diffraction limited on a typically widefield detector (e.g. a CCD camera). Many images of this kind are generated following each other. In each image the center of mass of each and every individual molecules are calculated and overlayed by an algorithm.

Further, PAINT and DNA PAINT are superresolution techniques wherein fluorescent molecules are in a balance between binding and non-binding to defined binding sites. As a result different candidates of the same fluorescent molecule bind after each other to the same binding site. This is another way to tag defined binding sites in a non-permanent way, i.e. this mimics also ,on' and ,off' states, while the labels are on average always fresh i.e. with maximum fluorescence yield.

STED technique is based on stimulated emission depletion. A red shifted laser beam is used additionally to the excitation beam. The so called STED laser is forced into a spatial light distribution with zero intensity in the middle of the spot. In this configuration all molecules outside the very center of the excitation area are switched off via stimulated emission. To this end, only fluorescent molecules in the very center can emit light thus increasing the effective resolution compared to the confocal (i.e. only excitation light present) configuration.

MINFLUX: the excitation light is shaped (similarly as a STED laser) into a light configuration with a zero intensity in the center (i.e. not gaussian shaped). This light configuration is iteratively moved, i.e. via circles with decreasing radius, close to each and every individual fluorescent molecule. Low fluorescence intensity similar to the background level on the detector indicates the correct position of the imaged molecule which is usually found out by triangulation as described in the pertinent publications on MINFLUX. This defines the spatial position of the individual molecule with very high precision, down to the molecular resolution level in three dimensions. Tracking of moving fluorescent molecules is also possible. The movement of the light zero as aclose as possible to the molecule mimics and thus enables recording of the spatial positions of the moving target molecule with unprecedented precision.

MINSTED: here a switchable or photoactivatable or a PAINT-binding dye is combined with a STED-microscopy arrangement as described by Weber et al, in "MINSTED fluorescence localization and nanoscopy", Nature Photonics 15, 361-366 and by Weber et al in bioarxiv, doi: https://doi.org/10.1101/2022.03.18.484906. Typically this results in even higher molecular resolutions.

In an embodiment of the method according to the present invention, the superresolution microscopy may be the method called dSTORM as amply described in the literature known to a person in the art of superresolution. In a further embodiment of the method according to the present invention, the superresolution microscopy may be PAINT (Point Accumulation Imaging and Nano Tomography) microscopy. The fluorescent markers are selected from fluorescent markers which are movable in the sample and individually attached to a predetermined structure in different positions or, alternatively, which are individually attached to different compounds at which interactions may be examined. These fluorescent markers are added to the sample in such a concentration and such a spatial distribution that there are sequentially and individually attached to the predetermined target. When they are attached to the predetermined structure, their position is determined according to the invention. Thus, in this embodiment of the method, the same individualisation of the fluorescent markers is used as in the PAINT concept. Correspondingly, all variants of this individualisation which are known for the PAINT concept may also be used in this embodiment of the method according to the present invention. This is particularly true for the specific embodiment of the DNA paint. Thus, the fluorescent markers may be transferred out of an inactive state in which they do not produce sufficient fluorescent light in an active state in which they are excitable with the excitation light for the detection of a significant amount of fluorescent light that is higher than the noise. This transfer may be due to the attachment of the fluorescent markers to the structure such well-known fluorophores are called fluorogenic. Alternatively or additionally, the fluorescent markers may only temporarily attach to the predetermined structure or compound or they are permanently or at least for such long time transferred in a dark state in which they do not disturb the measurement of the respective nano scale structure to the prolonged time influence of at least one of the excitation fluorescent lights.

In an embodiment, the method is a method comprising the steps of diffraction limited imaging, PALM/STORM superresolution microscopy as a first superresolution microscopy followed by the MINSTED and/or MINFLUX superresolution microscopy. The additional step of MINFLUX and/or MINSTED superresolution microscopy on the basis of the PALM/STORM superresolution microscopy enables a more accurate determination for the properties of the components of interest and, in addition, speed up the high throughput screening of drug candidates. Hence, combining the three technologies in one process improves high throughput superresolution drug screening accordingly.

Further, the present invention refers to a database containing imaging and tracking information and data of known components. Thus, a comparison of the data and information obtained with the method according to the present invention and the data and information available from a database allows for information on the compounds of interest. The database may form part of the system of the present invention or may be available remote. Further, based on the data and information available from the database and artificial intelligence, the process can be shortened and the imaging data determined in the method may be preselected reducing the data set.

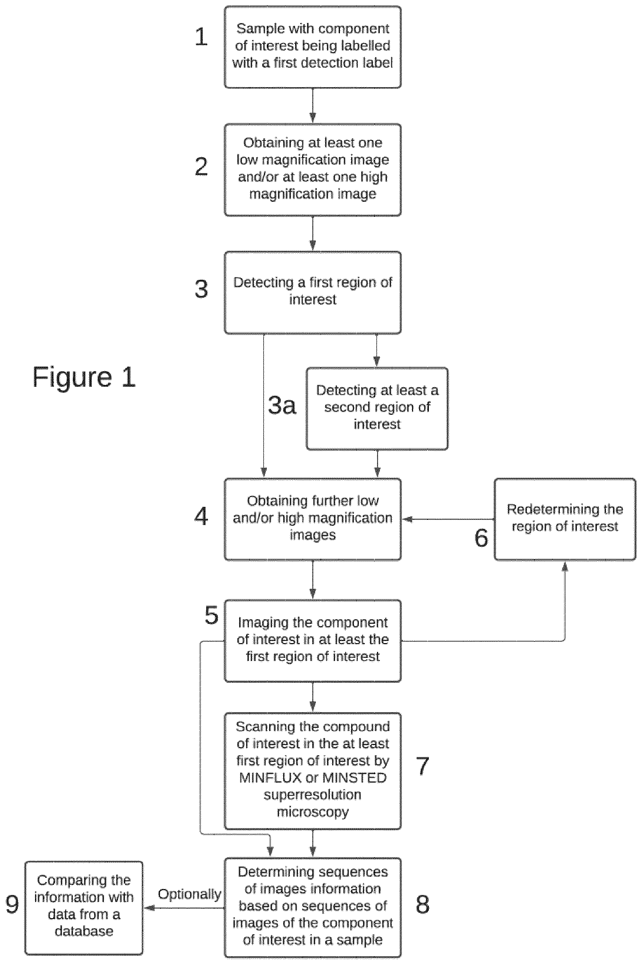

In the following, the method is described by reference to the drawings. In figure 1, the method is described in a flow chart starting with the step of providing a sample with the component of interest, whereby said component of interest is labelled with a first detection label. As identified herein, the sample may be labelled with at least a further detection label, not shown. For example, in drug screening methods and, in particular, with respect to high throughput drug screening, the candidate compounds are labelled with the first detection label, while structural elements of the sample, in particular, nanoscale structures, are labelled with at least a second detection label or, in addition or alternatively, other externally provided compounds are labelled with at least a further detection label. The detection label differ in their spectral properties with respect to at least one of the excitation lights by which they are excitable for the emission of fluorescent light and the fluorescent light emitted by the following to the excitation.

A step of obtaining at least one low magnification image or at least one high magnification image as step 2 is shown in figure 1 as a step following step 1. The image is typically obtained by diffraction limited technique as described herein.

In the next step 3, a first region of interest is determined, said first region of interest is determined based on at least one of the detection label or detection marker at the component of interest and/or the further detection marker and, optionally based on structural pattern of said sample. Particularly, the first region is determined by using of intelligent algorithms, including artificial neural networks as described herein.

Optionally, at least a second region of interest is determined 3a as described, in particular based on data and information processed by computer and/or artificial intelligence.

In step 4, further low magnification images and/or high magnification images are obtained by a device, typically, a microscope based on diffraction limited imaging method in the form of additional or further images, wherein the component of interest and other labelled components or structures are determined by of image sequences wherein according to step 5 the component of interest is imaged in at least the first region of interest in the images obtained in step 4.

Based on the imaging results, optionally redetermination of the region of interest or the further regions may be performed in step 6, thus, determining the first and second region of interest and possible further regions of interest.

Based on the imaging results, redetermination of the region of interest or the further regions may be optionally performed in step 6, thereby, determining the first and second region of interest and possible further regions of interest.

Based on the obtained imaging results, the regions of interest are determined and, in the next step 7, the screening of the component of interest is performed in at least the first region of interest by MINSTED and/or MINFLUX superresolution microscopy. This screening includes sequences of images that allows to determine the movement and tracking of the component of interest and to observe dynamics in reaction and interaction.

In step 8 the data and information obtained based on the image sequences of the component of interest in the sample including the movement and interaction as well as reaction with other compounds and components present in the sample are determined and, optionally the determination comprises the additional step of comparing the information with data from a corresponding database. By comparing the obtained data and information for the component of interest with the information in the database 9, it is possible to make an early and rapid decision on the suitability of the components of interest for specific purposes.

In addition, as shown in figure 1, the imaging data of step 5 may be used without the additional steps of superresolution imaging for data comparison with data sets in a database. Shortening of the process and reduction of the data set is possible.

That is, an "in- our out" decision for the compounds is possible in drug screening by high throughput screening, based on additional information obtained from the method according to the present invention and, in particular, compared with the data available in the database for known compounds or known interactions which also inlcude information on particular activities with respect to diseases, disorders or conditions etc.

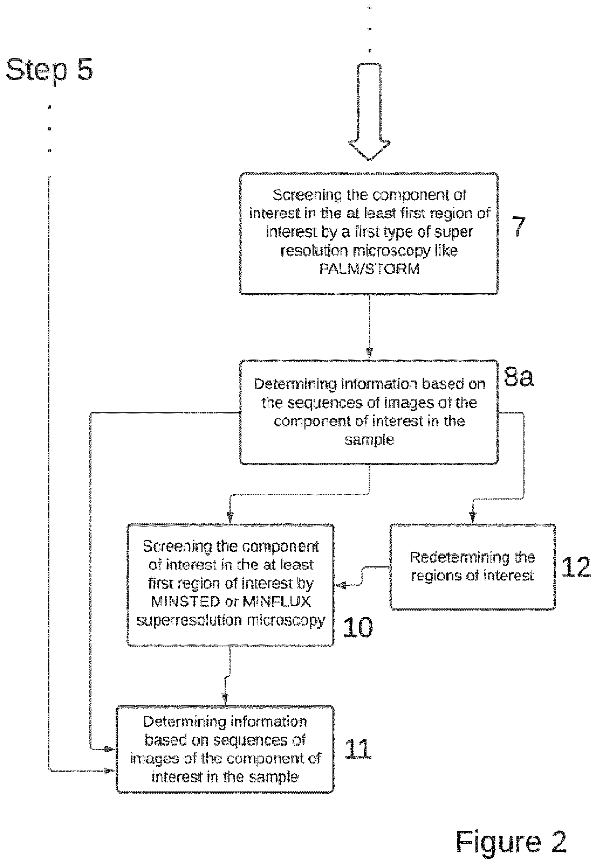

With reference to figure 2, an embodiment of the present invention is described. Therein, the step of screening the component of interest by high resolution is conducted in step 7 for at least the first region of interest by a first type of superresolution microscopy. This type of superresolution microscopy may be e. g. PALM/STORM-based superresolution microscopy for localisation of components of interest in a region of interest with lower resolution. This screening is followed by the determination of information based on the sequences of images of the component of interest by the first type of superresolution microscopy, step 8a. Here, a large number of components of interest can be screened and information obtained, that allows for further selection of specific components of interest which may then be further analysed by a second type of superresolution technology. In addition, the data set and the information of step 5 allows for rapid processing leaving out superresolution imaging in each iteration of the method. That is, according to step 10, further screening of the component of interest in the at least first region of interest is conducted by a second type of superresolution microscopy. This superresolution microscopy is preferably a type of superresolution microscopy with higher nanoscale resolution. For example, the second type of superresolution microscopy is MINSTED or MINFLUX.

Screening allows to obtain further information, like movement, tracking of the components of interest as well as their interaction with other structural patterns or components present in the sample to be analysed 10. Optionally, as described in figure 1, a comparison step is conducted with data and information available in a database. However, the screening may not be performed every cycle but, as described above, the screening step by the first or second superresolution may be performed every fifth, tenth or the like and the imaging with low and/or high magnification may be sufficient. Namely, each of the imaging or screening or tracking may have its own rate. Hence, the database as described herein contain dataset of components based on the different types of imaging. In a further embodiment of the present invention, the MINFLUX and/or MINSTED superresolution may be performed in initiating cycles only until determining the mechanism of action, followed by conducting the diffraction light imaging and/or first superresolution step and the determination of image sequences information only. Thus, the data set is reducing while maintaining the advantages of the MINFLUX and/or MINFLUX technique. Hence, data and time saving steps are possible without losing accuracy of the superresolution, namely the information e.g. on colocalization of molecules as detailed herein.

The information collected with the method according to the present invention and being part of the database include colocalization of molecules, e.g. receptor as reference signal and the compound of interest as the second signal; clustering vs. homogeneous distribution; number of clusters, density and/or size of clusters; protein-protein interaction; i.e. tracking of two individual molecules respectively to each other.

With the specific embodiment described in figure 2, it is possible to benefit from the two different types of superresolution microscopy. For example, with the first superresolution microscopy technique is PALM/STORM, tracking and imaging of large numbers of individual compounds is possible (typically hundreds or more), however, low nanoscale resolution is given (less than 10 nm). With the second superresolution microscopy technique MINFLUX or MINSTED, higher resolution of specific, predetermined compounds is possible, allowing a better understanding of interaction and movement as well as reaction with components and compartments in the sample.

It is particularly preferred, that the different steps can be performed with a single device, namely, a single microscope.

As said, artificial intelligence, intelligent algorithms, like artificial neural networks, can be used for determining the regions and compounds accordingly.

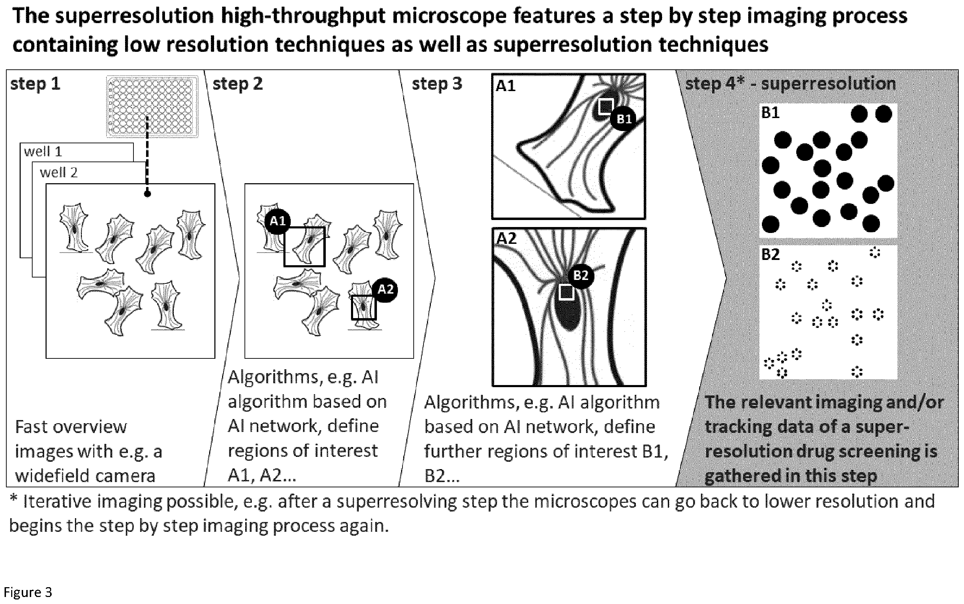

In figure 3 the steps according to the method according to the present invention are detailed further, i.e., the superresolution high throughput microscope has a step by step imaging process that includes low resolution techniques as well as superresolution techniques.

Without repeating the description of figure 1, in step 1 a quick overview of the images is performed using diffraction limited techniques, here a widefield camera is mentioned. In step 2 the regions of interest are determined, e. g. by algorithms defining regions of interest a1, a2, and so on. As shown, the sample is labelled by a further label, here two structures of interest are labelled: (i) a cytoskeleton protein, e.g. tubulin or vimentin (long strands within each cell) and (ii) a protein in the cell nucleus. Only the superresolution image in step 4 can identify differences in the protein distribution; in B1 the labelled protein clusters in individual spots/clusters without substructure; in B2 the labelled protein is arranged in a 6-fold symmetric substructure.

In the next step, at least second regions of interest are defined with applying algorithms, e. g. artificial intelligence algorithms, based on AI networks for further imaging in high resolution, namely, superresolution techniques.

Step 4 identifies the superresolution, allowing imaging and/or tracking of the components of interest. Namely, imaging and tracking data is obtained for the components of interest, e. g. in the case of drug screening, showing the specific movements, interactions, etc. of the components accordingly. As noted, an iterative imaging is possible. That is, iterative imaging may involve repeating step 1 to step 3 of starting with lower resolution and determining and defining the regions of interest accordingly. Of course, also the information and data obtained in step 2 and 3 can be compared with data available from a database.

Based on this technique, high throughput screening in drug discovery can be improved. In particular, earlier and faster decisions on favourable and non-favourable substances is possible, saving screening time and costs. Moreover, better decisions can be made about substances and optional chemical modification, leading to improved drug development results with better dedicated effects on the targeted disease or event. Indeed, with the method according to the present invention more detailed and, thus, superior information about the respective components can be obtained in form of super resolution and/or tracked data.

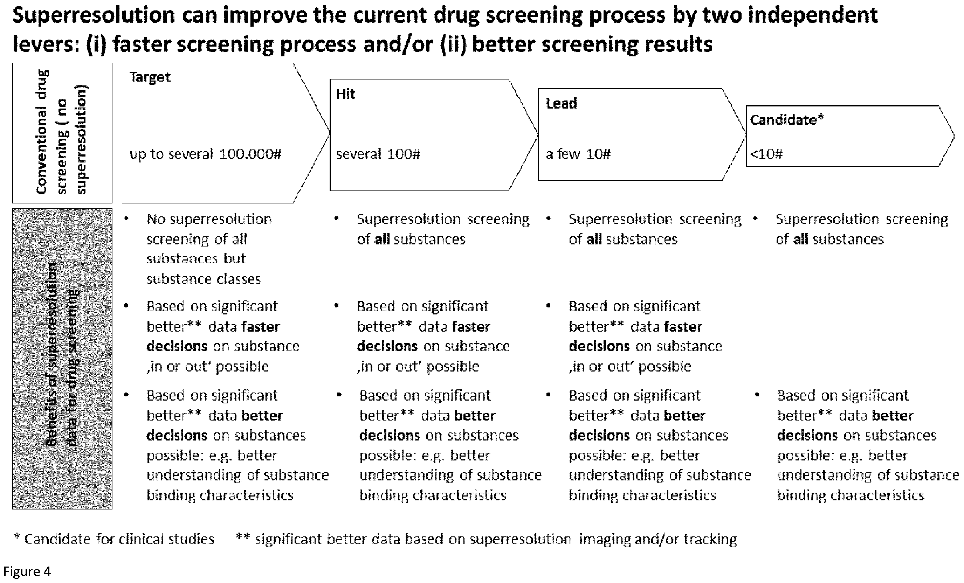

Figure 4 summarizes the improvement obtained by combining low magnification and superresolution microscopy in current drug screening processes. As shown, compared to the conventional drug screening chain, the combination of both low and superresolution microscopy allows the drug screening process to be accelerated, in particular, avoiding multiple steps, starting with the target, identifying hits which are further screened in a second round, and lead to lead molecules that eventually results in candidate and candidate molecules. In contrast, the combination of the drug screening with superresolution data is advantageous for analysing targets and hits even lead to the identification of lead compounds in a single round, as the process is faster and, in view of the superresolution data, earlier decisions on the substances as favourable substances for the desired purposes can be done. Moreover, the superresolution data allows the provision of data on interaction with other components present in the sample, either present naturally in the sample or added externally for determining the desired properties eventually treating the target disease.

The superresolution data obtained and the resulting information can provide information on tracking movement on the compound of interest in a region of interest, interactions with other components present in the sample but also externally provided compounds, colocalization of molecules, e.g. receptor as reference signal and the compound of interest as the second signal; clustering vs. homogenous distribution; number of clusters, density and/or size of clusters; protein-protein interaction, i.e. tracking two individual molecules respectively to each other.

In an embodiment, the method according to the present invention, a desired property of the compound of interest is determined based on the obtained data, in particular, by comparing the sequence of images information obtained with corresponding information from a database. Said database may contain corresponding information from known components or targets etc.

In an embodiment of the present invention, the method includes a predetermined selection of regions of interest or an automated or semi-automatic selection of regions of interest based on the selected component of interest and the selected possible properties. For example, in case of identifying molecules that interact with DNA or compounds involved in DNA transcription or regulation, including methylation, etc., the predetermined selection by automated or semi-automated computer generated tools, include identification of nuclei in a cell, e. g. based on structural patterns on respective label.

In an embodiment of the present invention, the sample is a living material including cells or organisms. In particular, the present invention relates to a method of obtaining time tracked superresolution data in living materials, thus, which has benefits in drug screening, in particular, identifying suitable compounds.

In another aspect, the present invention provides a system for image sequences imaging and screening a component of interest in a sample comprising a microscope system being an optical microscope combining diffraction limited imaging techniques allowing low magnification images and high magnification images, and superresolution techniques including at least one of PALM, STORM, single module localization microscopy, PAINT, DNA PAINT, and STED for a first superresolution microscopy of low nanoscale resolution. This is followed by the second superresolution microscopy of MINFLUX or MINSTED, a data processing unit comprising a software or a computer program product for carrying out the method according to the present invention, optionally a database contain imagining and scanning data wherein the data processing unit containing a hardware processor being configured to perform operations as cited in the method according to the present invention. The system according to the present invention is suitable for use in high throughput screening, in particular, drug screening for a method defined herein. In particular, the system comprises a microscope system with corresponding units for diffraction limited imaging, including detector units, allowing low and/or high magnification images. In addition, the microscope includes units that enables superresolution techniques as defined herein, including at least one of PALM/STORM, single module localisation microscopy, PAINT, DNA PAINT, and STED in combination with a unit for MINFLUX or MINSTED. In an embodiment of the present invention, the microscope system of a system according to the present invention includes at least two units for superresolution microscopy, including at least one unit allowing MINFLUX or MINSTED.

Additionally, a data processing unit and, optionally, an output unit is present. Said data processing unit comprises computer assisted processing of the raw data obtained, i.e., the scanning and tracking by superresolution technique and the images obtained by diffraction limited imaging techniques. The data processing units determine information about the compounds of interest with respect to dynamic movement, tracking, interaction, etc., of the compound of interest with other components present in the sample. Moreover, time resolved and spatially resolved data of the components of interest in absolute terms or relative to other components are determined in the sample. Optionally, a database is present for connections allowing for connecting with a database, said database contains imaging and scanning data. The processing unit or a separate data processing unit may compare the data and information measured with the system with data and information present in the database to obtain further information on the compound of interest. The data base may be part of the system or is accessible remotely. Further, in the data processing unit present in the system according to the present invention contains a hardware processor configured to perform operations according to the present invention. In particular, the data processing unit comprises intelligent algorithms, like artificial neural network, for processing the data according to the present invention.

In an embodiment of the present invention, the system is configured to determine multiple fluorescent dyes on one target, allowing sequences of images imaging and/or tracking of the one target with different imaging and scanning methods. That is, while low imaging or imaging with a first superresolution technique, identifies and tracks the component of interest, a further superresolution technique having higher resolution allows identifying interaction and tracking of specific components of interest with unprecedented precision.

In an embodiment of the present invention, the microscope comprises a first imaging system for diffraction limited techniques and at least a second imaging system for superresolution by MINFLUX and/or MINSTED, including laser.

Further, the system according to the current invention or the method according to the present invention is particularly useful for drug screening, in particular, high throughput drug screening, as described above.

Finally, the present invention provides a computer program product residing on a computer readable medium with a program code that when it is running on a computer or was loaded onto a computer, causes the computer to perform a method according to the present invention for controlling a microscope system.

Regarding the laser and the laser beams of the system according to the present invention and the use in the method according to the present invention, in an embodiment, the laser beams are focused beams and can be aligned with each other. Hence, it is possible to obtain full information from all imaging and/or tracking methods spatially directly contacting each other within one measurement.

In an embodiment, the individual lasers to be used according to the present invention can be pulsed or CW or both, that is, a laser switchable between both radiation modes may be used. For MINFLUX or MINSTED the same laser beam may be used, e. g. a pulsed beam of 1 ns pulsewidth.

In an embodiment, the laser beams in the system can be combined in one optical fiber. In addition, it is possible that all laser beams can be generated by one laser source.

When using the STED technology, one or more STED channels may be used, in case of using the MINFLUX technology, one or more MINFLUX imaging channels may applied. Moreover, when applying the MINFLUX technology, one or more tracking channels may be used. The microscope present in the system according to the present invention or constituting the device according to the method in the present invention may contain a conventional microscope body and other modalities like DIC, transmitted light detection or others. These modalities may be used to generate further data and eventually information about the sample which may also be part of the information present in the database.

The microscope itself can contain an auto alignment routine. In addition, the microscope may contain a dispenser for an oil and/or water and/or other emersion media.

Moreover, the microscope may have hardware and/or software autofocus to compensate for the system drifts known to the skilled person.

Moreover, the microscope may have hardware and/or software autofocus to compensate for the system drifts known to the skilled person.

The algorithms used in the method or underlying the computer program according to the present invention can be an artificial intelligence algorithm based on an artificial intelligence network. In particular, the network is trained for the particular functionality. The algorithm may be fully automated independent or semi-automated. That is, the user has to interact with the algorithm to process the data properly. In case of fully automated selection, the regions of interest can be optimized for drug screening use case. Of course, this is also possible in semi-automated selection. Moreover, the software and the method may comprise uses of a set of algorithms for the automated or semi-automated processing, e. g. for statistical analysis of imaging or tracking results. In particular, a self-learning algorithm for further optimizing the method and selection of regions of interest.

In a further aspect, an assay preparation is possible with multiple dyes on one target, namely, on a compound or component of interest allowing imaging and/or tracking of the one target with multiple modalities. These multiple modalities may include the use of confocal microscopy on the one hand, combined with STED and/or MINFLUX on the other hand.

In addition, an assay preparation is possible with multiple dyes on multiple targets or compounds or components of interest, allowing multiple targets imaging or tracking with different modalities. These different modalities may be a combination of any one of the above referenced types of superresolution techniques or diffraction limited techniques.

The assay preparation may be in form of a kit of parts, including the necessary agents for coupling the dyes to the target. As noted, the assay preparation may involve the use of CLICK chemistry or other types of linking label molecules to the components of interest. In addition, the assay preparation may include suitable solvents and buffers for conducting linkage accordingly.

The present invention identifies kit of parts which may contain additionally other equipment required for performing the method according to the present invention including suitable well plates etc.

The method according to the present invention is particularly useful for drug screening in living materials, including cells or organisms. As described above, *in situ* and *in vivo* tracking and imaging is possible at high nanoscales, like with a resolution of below 5 nm. It is possible fast and easy determination of components of interest for allowing better decisions on hits, leads and candidates in drug screening. In particular, the method according to the present invention allows for perform high throughput resolution drug screening within one step, speeding up the process accordingly.

## Claims

1. A method of image sequences imaging and screening a component of interest in a sample, comprising the steps of:
a) Providing a sample, like living material, with the component of interest whereby the compound of interest is labelled with a first detection label and, optionally, the sample is labelled with at least one further detection label;
b) Obtaining at least one low magnification image and/or at least one high magnification image of the sample by diffraction limited imaging with a device;
c) Determining a first region of interest of said sample based on the low and/or high magnification image of step b);
d) Optionally determining further at least one second region of interest of said sample based on the low and/or high magnification image of the sample obtained in step b);
e) Obtaining and imaging the component of interest in at least the first region of interest by low and/or high magnification imaging by diffraction limited imaging;
f) Optionally redetermining at least one of the regions of interest;
g) Screening the component of interest in the at least first region of interest by MINSTED or MINFLUX superresolution microscopy;
h) Imaging and/or tracking the component of interest in the sample and determining information of the component of interest in the sample based on steps e) and g), in particular, image sequences information.

2. A method of image sequences imaging and screening a component of interest in a sample according to claim 1, wherein the diffraction limited imaging is light sheet, widefield, confocal, structured illumination, or epifluorescence microscopy.

3. A method of image sequences imaging and screening a component of interest in a sample according to claim 1 or 2, comprising further the step f1) of scanning the at least first region of interest and the component of interest by PALM/STORM, single molecule localization microscopy, PAINT, DNA PAINT, or STED before screening the component of interest in the at least first region of interest by MINSTED and/or MINFLUX of step g) and conducting step h) based on the screening of step f1.

4. A method of image sequences imaging and screening a component of interest in a sample, wherein the method is under control of a computing device, including the further step of:
i) a computer based identification of one of more regions of interest within the low magnification or high magnification image of step b) or step e).

5. A method of image sequences imaging and screening a component of interest in a sample according to claim 4, wherein the computing device includes an intelligent algorithm, e. g. artificial neural network, and the identification of the one or more regions of interest is based on the artificial neural network.

6. A method of image sequences imaging and screening a component of interest in a sample according to any one of the preceding claims, comprising tracking the movement of the component of interest in a region of interest, determining possible interaction with other components present in the sample including externally provided components.

7. A method of image sequences imaging and screening a component of interest in a sample according to any one of the preceding claims, wherein i) steps e) to h) are repeated at least once, and/or ii) step e) is iterated, alone or in combination with step f1), followed by step h) to image and/or track the component of interest..

8. The method of image sequences imaging and screening a component of interest in a sample according to any one of the preceding claims, wherein steps e), f1) and g), each in combination with step h), are iterated, optionally, with different rates, to determine the mechanism of interest, followed by iterating steps e) and/or f1), each in combination with step h) for imaging and/or tracking the compound of interest.

9. A method of determining a predetermined property of a candidate component in a sample, like in living materials, including the method steps of any one of claims 1 to 8, further comprising the step of:
j) Analysing the predetermined property of said candidate compound based on the scanning and imaging data obtained,
k) Optionally, comparing the information with data from a database,
in particular, wherein the candidate molecules are candidate drug molecules and the predetermined property is a drug property on components of the sample, like in the living material, in particular, the method is a method for drug screening, like comprising determining interaction of the candidate molecule with predetermined other components present in the sample or determining a time-resolved localisation movement of the predetermined component.

10. A system for sequence of images imaging and screening a compound of interest in a sample, comprising a microscope system being an optical microscope combining diffraction limited imaging technique allowing low and high magnification images, and superresolution technique, including at least one of MINSTED, MINFLUX, a data processing unit comprising a software or computer program product for carrying out the method according to at least one of claims 1 to 9, optionally, a database containing imaging and scanning data, wherein the data processing unit contains a hardware processor being configured to perform operations assigned to the method according to any one of claims 1 to 9 for use in drug screening and drug discovery.

11. The system according to claim 10, configured to determine multiple fluorescent dyes in living material allowing time resolved imaging and/or tracking of the target with different imaging and scanning methods and/or wherein the microscope comprises a first imaging system for diffraction limited imaging techniques and at least a second imaging system for superresolution by MINFLUX or MINSTED, including laser.

12. The system according to any one of claims 10 or 11, comprising a database containing imaging and scanning data of component s displaying the predetermined property to be determined and means for comparing the imaging and scanning data present in the data base with the imaging and scanning data obtained with the system according to any one of claims 10 or 11.

13. The system according to any one of claims 10 to 12, comprising an intelligent algorithm, like an artificial neural network, for training the system in a reinforcement learning scheme.

14. The system according to any one of claims 10 to 13 for use in high throughput screening of drug candidates, in particular, for drug discovery.

15. Computer program product residing on a computer readable medium with a program code when it is running on a computer or was loaded onto a computer causes the computer to perform a method according to any one of claims 1 to 8 for controlling a microscope system.

16. A kit of parts comprising agents for labelling components of interest including coupling the label to the components, suitable container or carrier for the sample, like well plates for superresolution screening, microfluidic devices, lab-on-a-chip devices, robotically controlled devices.
